Europäisches Patentamt

**European Patent Office**

**Office européen des brevets**

(19)

(11) Publication number: **0 395 223**
**A2**

# EUROPEAN PATENT APPLICATION

(12)

(21) Application number: **90303301.7**

(22) Date of filing: **28.03.90**

(51) Int. Cl.5: **A61F 13/46, B65D 81/26**

(30) Priority: **24.04.89 US 343812**

(43) Date of publication of application:
**31.10.90 Bulletin 90/44**

(84) Designated Contracting States:
**DE FR GB IT**

(71) Applicant: **AMERICAN COLLOID COMPANY**
**One North Arlington, 1500 West Shure Drive**
**Arlington Heights, Illinois 60004(US)**

(72) Inventor: **Alexander, William**
**242 Terrance**
**Naperville, DuPage, Illinois(US)**
Inventor: **Hughes, John**
**2719 N. Brighton Place**
**Arlington Heights, Cook, Illinois(US)**
Inventor: **Perkins, Daphne A.**
**5843 Washtenaw Street**
**Chicago, Cook, Illinois(US)**

(74) Representative: **McCall, John Douglas et al**
**W.P. THOMPSON & CO. Coopers Building**
**Church Street**
**Liverpool L1 3AB(GB)**

(54) **Pouch for absorbing fluid.**

(57) There is disclosed a pouch (20) for absorbing fluids such as water, alkaline and acidic liquids, organic liquids, saline solutions, urine, blood and other body fluids comprising an inner layer of liquid-insoluble, liquid-absorbing polymer material (26) sandwiched between a pair of thin, resilient flexible outer, liquid-permeable sheets (28) for passage of liquid from the exterior of the pouch inwardly therein to contact and be absorbed by the liquid-absorbing material of the inner layer. The thin sheets may comprise a single sheet folded over on itself. The outer sheets are formed of water-permeable, flexible, woven or non-woven, fabric or polymeric sheet material. In one embodiment, the outer layers have sufficient wet strength and include resilient, thermoplastic, essentially non-absorbent fibres, along with liquid-absorbing, fibrous material. The fabric sheets are secured together around peripheral edges for containing the liquid-absorbing polymer material and fluids absorbed thereby.

FIG_2

# POUCH FOR ABSORBING FLUID

The present invention relates to a new and improved pouch for absorbing fluids such as water, alkaline and acidic liquids, organic liquids, saline solutions, urine, blood and other body fluids and a wide variety of other types of liquids and chemicals. More particularly, the pouch is designed to accompany a wide variety of different types of packages and/or containers used for carrying, holding and transporting various types of fluids so that in the event the containers leak or rupture, any fluid spilling out will be promptly absorbed with minimal damage to the immediate environment.

The pouch in accordance with the present invention is especially designed and adapted for absorbing contaminated body fluids such as urine, blood and other types of liquids used, or obtained for analysis, in medical treatment.

In addition, the pouch finds commercial utility in packaging for meats and other foodstuffs for absorbing liquids and fluids that may be secreted or released from food. The pouch is useful in packaging to insure a neat and clean appearance of a package free from blood stains and the like, which stains are sometimes present in the packages of meats, poultry, fish and other foodstuffs.

Over the years a great deal of progress has been achieved in providing new and improved fluid absorbing media in items-such as diapers, sanitary napkins, incontinent garments and industrial spill absorbing ropes, pigs, and the like.

The following U.S. patents disclose various types of fluid and liquid absorbing devices. DePhillips, Patent No. 2,527,299 discloses a water impermeable sheet material which is capable of passing vapors therethrough. Hoey Patent No. 3,901,240 discloses a permeable polymeric liner on an absorbent pad of foam which disintegrates upon flushing in a sewage system. Kemp Patent No. 3,953,638 discloses a multi-ply, absorbent, wiping product having a relatively inextensible inner ply that is bonded to highly extensible outer plys. Burkholder Jr. Patent No. 3,959,569 discloses a process for preparing water absorbent articles such as bandages and absorbent pads.

Hoey Patent No. 4,069,366 discloses a permeable polymeric liner for absorbent pads used in sanitary napkins, wound dressings and the like. Holst et al Patent No. 4,200,558 discloses a process for producing hydrophylic articles from water insoluble polymers. Erickson Patent No. 4,293,609 discloses a flexible absorbent laminate made from a crushed film of lightly cross-linked or water soluble hydrophylic polymers. Fowler Patent No. 4,372,309 discloses a moisture absorbent pad of wood pulp impregnated with a starched polymer or an acrylic based polymer and useful in absorbing liquid wastes such as urine and the like.

Wahlquist et al Patent No. 4,379,192 discloses a non-woven, melt blown, absorbent barrier fabric useful in hospital projects such as surgical drapes and the like. Dehnel Patent No. 4,392,908 discloses a process for making water-absorbent articles having water-soluble polymers fixed to a water-absorbent substrate. Dawn et al Patent No. 4,411,660 discloses an absorbent product for body wastes such as urine and fecal matter employing multiple layers of material for passing and absorbing fluids and liquids, and having a layer for contact with the skin which is leak-proof. Michaels Patent No. 4,223,061 discloses an internal body drug dispenser using liquid swellable material to expand and disperse the drug.

Korpman Patent No. 4,449,977 discloses a flexible, non-disintegrative, absorbent product having a water-insoluble, non-swelling matrix. Hag et al Patent No. 4,603,069 discloses a sheet-like article for wiping hard surfaces and the like having an inner layer of liquid absorbing material and bonded together with spot welds between outer sheets. McFarland et al Patent No. 4,604,313 discloses a fabric-like material containing melt blown material and wood fibers deposited on a foraminous belt. Ness Patent No. 4,629,457 discloses an absorbent facing material and method for making the same having a significant one-way valve characteristic for aqueous fluids. Ito et al Patent No. 4,364,992, discloses a two-layer absorbent article having a super-water-absorbing polymer. Duncan Patent No. 4,701,369 discloses an opaque, polymer film laminate having an absorbent surface for absorbing and containing liquids, and Duncan Patent No. 4,702,954 discloses an opaque, barrier layer capable of transmitting a vapor therethrough.

It is the object of the present invention to provide a pouch for absorbing fluids and e.g. to provide a pouch for absorbing and containing fluids leaking from packaging, shipping containers and the like during shipment and/or storage.

The pouch of the present invention may be used for absorbing body fluids, urine, fecal matter, blood, and other body wastes and contaminants in a highly efficient manner to preclude contamination and damage to the immediate environment should liquid leakage occur.

By use of the invention one or more of the following may be achieved.

(i) a low cost, highly absorbent pouch of the character described which is capable of absorbing and holding quantities of liquid much in excess of the weight and un-swollen volume of the pouch itself,

(ii) a pouch of the character described which will not disintegrate and capable of retaining its original form and general shape even after absorbing a high volume of liquid,

(ii) a pouch of the character described which is relatively low in cost and yet still capable of absorbing many times its own volume and weight in liquids.

The foregoing object may be accomplished in a new and improved pouch for absorbing liquids comprising an inner layer of liquid-insoluble, super-liquid-absorbing-polymer material, sandwiched between a pair of thin, flexible liquid-permeable fabric or water-permeable polymeric sheets having permeations therein for the passage of liquid from the exterior of the pouch inwardly to contact and be absorbed by the super-liquid-absorbing polymer of the inner layer. The inner polymer material comprises a quantity of par ticulate, water-absorbing polymer and the exterior fabric or water-permeable polymeric sheets may comprise, woven/non-woven, resilient fibrous or film material preferably having both non liquid-absorbing fibers as well as liquid absorbing fibers for drawing the liquid into the water-absorbing polymer from outside the pouch, as known in the art.

The thin flexible, resilient fabric sheets are secured together adjacent peripheral edges by linear heat seals or by adhesives, such as adhesive strips, to contain the super-liquid-absorbing-polymer material therebetween. The super-liquid-absorbingpolymer may be dispensed or sifted onto an inner surface of a lower fabric or permeable polymeric sheet or may be dispersed in a thin, substantially uniform thickness layer between a pair of woven/non-woven, liquid-permeable sheets. The outer sheets are then pressed together to form a discreet sheet-like article which is placed in the interior of the pouch between the outer fabric sheets before the outer sheets are secured together by heat seals or adhesive along peripheral edges.

In accordance with another embodiment of the present invention, a pouch is formed by providing an inner layer of liquid-insoluble, super-liquid-absorbing polymer, such as a cross-linked polyacrylate, disposed in a thin, substantially uniform layer pressed between a pair of outer thin, flexible, fabric or water-permeable polymeric sheets, which outer sheets are secured together around peripheral edges after folding over along a fold line initially bisecting the sheets.

In accordance with one important embodiment of the present invention, the pouch is utilized in packaging foodstuffs of the type having liquids secreted or released therefrom such as meat, fish, poultry, and the like. A liquid-insoluble, super-liquid- absorbing, polymer is sandwiched between a pair of thin flexible, fabric or water-permeable polymeric sheets and the sandwich sheet then is placed under the food on a support material or package for direct contact with the underside of the food. Any liquid secreted or dispersed from the food is absorbed in the super-liquid-absorbing polymer and is normally hidden from view, thus eliminating unappetizing blood stains or other unsightly discolorations and the like from appearing in the package.

The invention will now be further described by way of example with reference to the accompanying drawings, in which:-

FIG. 1 is a perspective view of one side of a new and improved pouch constructed in accordance with the features of the present invention and shown in a thin, flat condition before any contact with liquid to be absorbed;

FIG. 2 is a fragmentary transverse cross-sectional view taken substantially along lines 2-2 of FIG. 1;

FIG. 2A is a fragmentary transverse cross-sectional view taken substantially along lines 2A-2A of FIG. 1;

FIG. 3 is an elevational view of the pouch shown after the pouch has enlarged in size and weight because of the absorption of liquid therein;

FIG. 4 is a fragmentary transverse cross-sectional view taken substantially along lines 44 of FIG. 3;

FIG. 5 is a perspective view of one side of another embodiment of a new and improved pouch constructed in accordance with the features of the present invention and shown in a thin, flat condition before any contact with liquid to be absorbed;

FIG. 6 is a fragmentary transverse cross-sectional view taken substantially along lines 6-6 of FIG. 5;

FIG. 7 is an elevational view of the pouch of FIG. 5 shown after the pouch has enlarged in size and weight because of the absorption of liquid therein;

FIG. 8 is a fragmentary transverse cross-sectional view taken substantially along lines 8-8 of FIG. 5;

FIG. 9 is a perspective view of yet another embodiment of a sheet of liquid absorbing material to be used in a pouch manufactured in accordance with the features of the present invention and shown in a thin, flat condition before any absorption of moisture has taken place;

FIG. 10 is a fragmentary, transverse cross-sectional view taken substantially along lines 10-10 of FIG. 9;

FIG. 11 is a perspective view of the sheet of FIG. 9 after folding in half to provide a modified form of pouch, and shown in a condition before the absorption of any liquid;

FIG. 12 is a fragmentary, transverse, cross-sectional view taken substantially along lines 12-12 of FIG. 11;

FIG. 13 is a fragmentary, transverse, cross-sectional view taken substantially along lines 13-13 of FIG. 11;

FIG. 14 is a perspective view of one side of still yet another embodiment of a pouch constructed in accordance with the features of the present invention and shown in a partially assembled condition and before any absorption of moisture thereby;

FIG. 15 is a fragmentary, transverse, cross-sectional view taken substantially along line 15-15 of FIG. 14;

FIG. 16 is a fragmentary, transverse, cross-sectional view similar to FIG. 15 illustrating the pouch in a swelled condition after the absorption of liquid; and

FIG. 17 is a graphical representation of the liquid absorbing capability of a pouch in accordance with the invention over time commencing with the initial contact with the liquid.

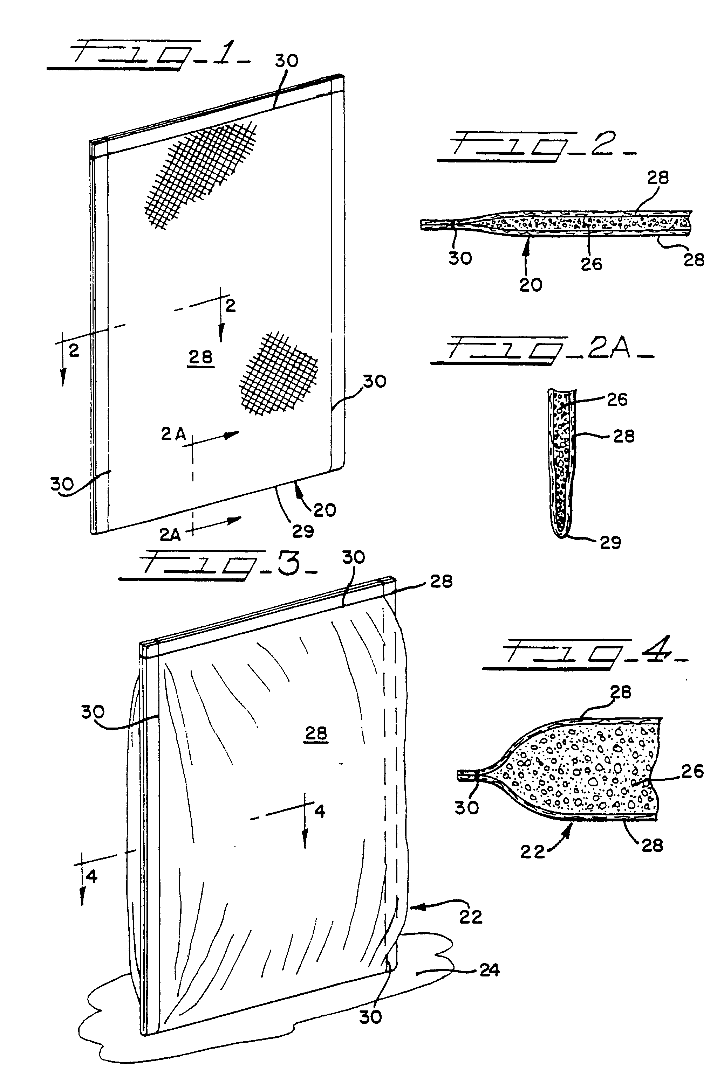

Referring now more particularly to the drawings, in FIGS. 1 through 4 there is illustrated a preferred embodiment of a new and improved pouch for absorbing fluids, referred to generally by the reference numeral 20 and especially adapted and designed to absorb and contain a relatively large volume of liquid 24 coming in contact therewith. The pouch 20 is especially well adapted for the absorption and containment of a wide variety of liquids such as alkaline and acidic liquids, organic liquids, industrial chemicals, water, aqueous solutions, saline solutions and body fluids of all types including urine, blood, fecal matter and the like, as well as contaminated medical fluids which may be shipped in packages and containers, and stored for periods of time.

In the event of spillage and/or leakage of these fluids from packages and containers during storage, handling or transit, it is highly desirable and mandatory in some cases, that the leaking fluids be absorbed immediately without any spreading of liquid into adjacent containers or cartons, and without contaminating or desanitizing the immediate environment.

The pouch 20 is also especially adapted for containing water and chemical spills, as well as aqueous solutions of acid and alkaline mixtures so that deterioration of the immediate environment where the spills or leakages occur, is minimized or eliminat ed altogether. Moreover, the pouch 20 is well suited for use in body garments for humans and animals for collecting and containing fluids and liquids that are secreted and released from the body. In addition, the absorption of these fluids in the pouch 20 is accomplished in a manner that results in a minimal amount of discomfort and in a manner which minimizes the wetness of the collected liquids on the skin or surface of the human or animal involved. The pouch 20 can be especially formulated for protecting the environment against leakages and spills of a wide variety of industrial chemicals, paints, solvents and other fluids and liquids which normally move in commerce and industry and which, if uncontrolled, upon leakage or spillage will cause grave damage and deterioration to the environment.

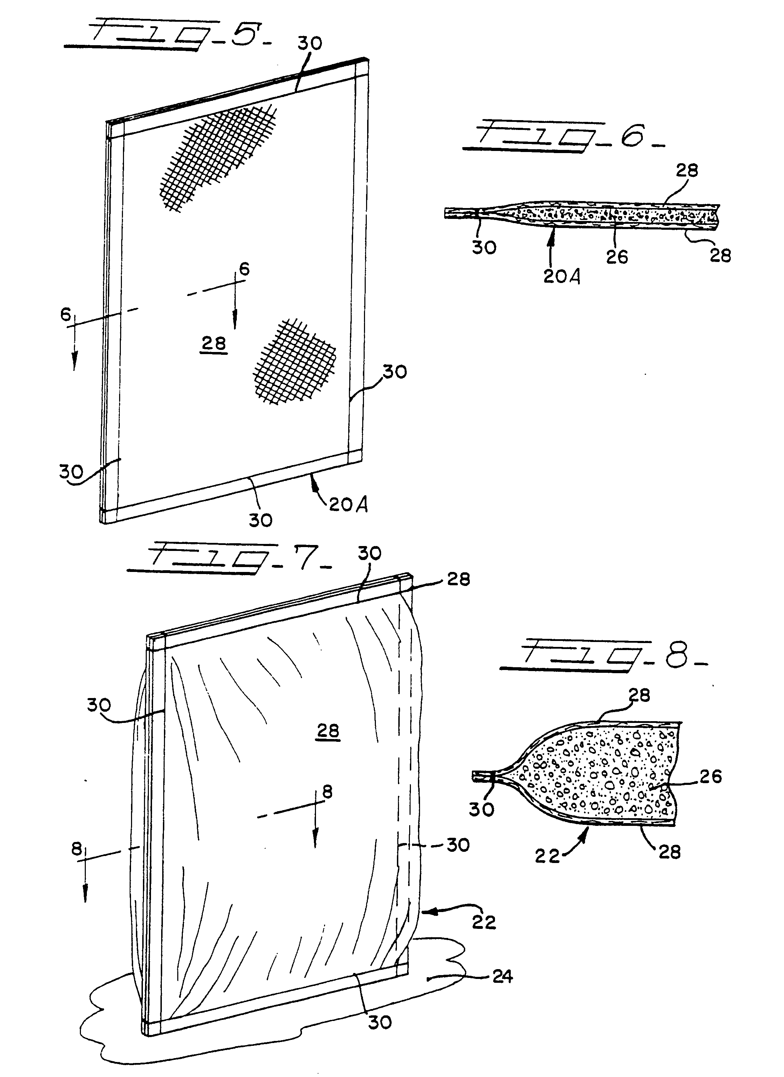

Referring now more particularly to FIGS. 1 and 2, the pouch 20 comprises an inner or inside layer of fluid insoluble, super-liquid-absorbing, polymer material 26, preferably in powdered or particulate form and contained in a layer, disposed between folded over half segments of a flat, thin, flexible resilient, fabric or water-permeable polymeric sheet 28 joined together along three (3) peripheral edges by means of linear heat seals 30 and/or suitable strips of adhesive material. Heat seals are formed by compressing the fabric sheet halves 28 tightly under heat and pressure to form heat seals 30, suitable to melt and bond thermoplastic fibers or thermoplastic sheet material 28 to provide a permanent line of seal. The sheet material 28 need not contain thermoplastic fibers if the sheet halves are adhesively secured together or if they are thermoset during manufacture.

In manufacturing the pouch 20, a relatively thin layer of super-liquid-absorbing, polymer material 26 in particulate, powdered, or granulated form is sifted onto approximately one-half of a rectangular piece, flexible fabric sheet 28 to a substantially uniform depth except for small strips forming narrow margins around the peripheral edges. The uncovered opposite half portion of the sheet 28 is then folded over along a fold line 29 to lie on top of the thin layer of super-liquid-absorbing polymer. Heat sealing is then accomplished along three (3) linear heat seal lines 30 by the application of heat and pressure to complete the closure or sealing of the pouch 20 containing the desired weight and thickness of the super-liquid-absorbing polymer 26, as illustrated in FIG. 2.

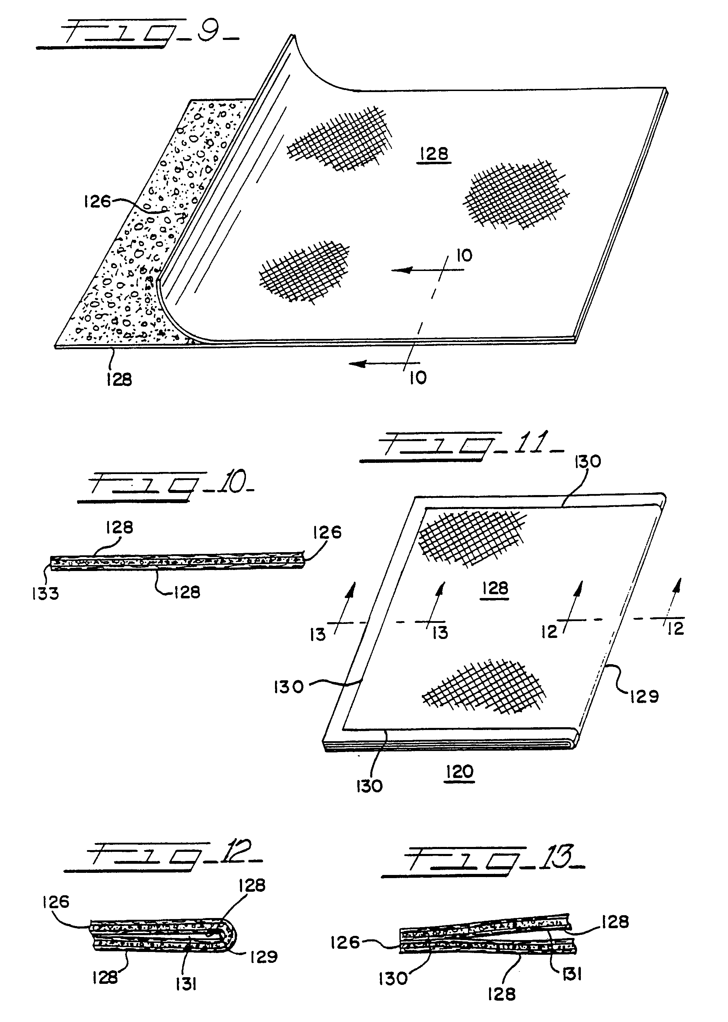

Subsequently, when the pouch 20 comes in contact with a liquid spillage or leakage 24, as illustrated in FIG. 3, the liquid is drawn through the permeable outer fabric or polymeric sheets 28 into the interior of the pouch for direct contact with the granules or particles or powdered super-liquid-absorbent polymer material 26.

Because the polymer is in particulate, granular or powdered form, a considerable amount of space is left in voids within the layer of polymer 26 between the sheet portions 28 so that liquid can move easily and freely throughout the entire thickness of the layer of super-absorbing, polymer 26 for rapid pick-up and absorption.

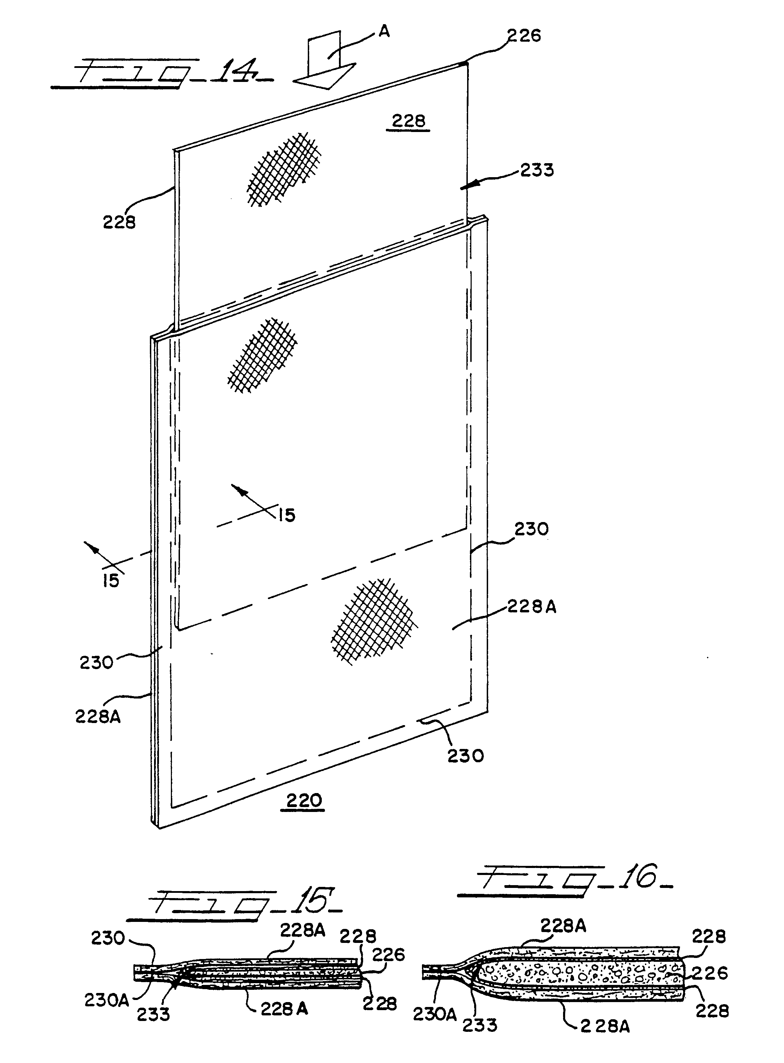

As the liquid is absorbed by the pouch 20, the polymer material 26 begins to enlarge and swell in size and forms a fluff or gel-like consistency. This swelling action expands the pouch volume from the interior as shown in FIGS. 3 and 4 as more and more liquid is absorbed. The super-liquid-absorbing-polymer 26 swells and enlarges the volume or space between the thin fabric sheet halves 28 to many times the original space or volume of the thin, flat, dry pouch 20 as shown in FIGS. 1 and 2. This increase in volume may run as high as 60 to 80 times the original volume and weight of a pouch 20 designed to absorb body fluids and petroleum, whereas, the wet to dry volume or weight ratio of liquid absorbed to dry weight may be as high as 800 times the weight of the super-liquid-absorbing polymer 26 when water or aqueous solutions are absorbed. Once the liquid has been absorbed, as illustrated in FIGS. 3 and 4, the liquid is retained and contained in the swelled super-liquid-absorbing polymer material 26 and does not tend to leak back out of the swelled pouch 22. In addition, the outer surface of the thin, water-permeable fabric or polymeric sheet halves 28 remains substantially dry to the touch and does not tend to dampen or wet a surface in contact therewith, such as a person's skin or the like.

Preferably, the pouches 20 are formed to be rectangular in shape, however a wide variety of different sizes and shapes can be made to fit a particular type of package or requirement. A wide variety of standardized sizes may be provided in order to accommodate standard package sizes or standard sized containers that are commonly used. For example, a prototype pouch 20 having a finished rectangular size of approximately 5" X 7" can rapidly absorb liquid 24 within a period of several seconds after initial contact with the liquid occurs. As illustrated graphically in FIG. 17, upon initial contact with a synthetic urine type of liquid, a very high absorption rate obtains initially and then the rate gradually reduces as time elapses and the absorbing polymer 26 approaches saturation. The graph of FIG. 17 illustrates by way of an ordinate or Y-axis the number of grams of synthetic urine liquid absorbed per each 20 grams of liquid-absorbing polymer 26 contained in the pouch 20. After only 15 seconds has elapsed, 271 grams of liquid have been absorbed by the pouch for each 20 grams of polymer therein and after 30 seconds have elapsed, 403 grams of liquid have been absorbed. The absorption rate then begins to level off and eventually 500 grams are absorbed after a minute and one half of time has elapsed since the initial contact with the liquid.

The pouch 20 is extremely light in weight and is thin and flat when dry as illustrated in FIGS. 1 and 2, and does not attribute to any appreciable increase in size or weight to a package containing liquids which are to be absorbed should they leak or containers rupture during transit, handling or storage. However, once a leak or rupture in a container does occur and the leaking liquid is absorbed by the super-liquid-absorbing polymer 26 within a pouch 20, the pouch swells rapidly in size and because of the flexibility and resiliency of the outer fabric or polymeric sheets, the swollen pouch tends to fill any voids or spaces in the package to prevent the liquid from sloshing around for any appreciable time after a rupture or leakage occurs.

A wide variety of super-liquid-absorbing-polymers 26 are useful in the pouch 20 and preferably these materials are supplied in powder form, in particulates or in suitably sized granules. In these forms, voids or spaces are present between the individual particles so that the liquid can freely come in contact with all of the volume of absorbent material in an efficient manner with little, if any, dry space being left within the interior of a pouch.

For pouches 20 designed to absorb spilled saline solutions, body fluids, blood, urine and other contaminated liquids, the aforementioned super-liquid-absorbing-polymer 26 may be selected from those capable of absorbing from 50 to 800 grams of fluid per gram of polymer material. If the polymers are in powdered form, to achieve the full advantage of the present invention, the material is sized to pass freely through a 20 mesh screen but be retained on a 100 mesh screen.

For fluids other than aqueous based liquids or body fluids, super-liquid-absorbing-polymers often have a capacity of absorbing approximately 50 to 600 grams of liquid per gram of polymer and these types of absorbents work well with fatty fluids, acidic and alkaline liquids and other pharmaceutical chemicals.

In particular for absorbing fluids from foodstuffs such as cut meats, including lamb, beef, pork, veal, poultry and fish, super-liquid-absorbing-polymers 26 often have a capacity to absorb approximately 50 to 600 times their own weight in such liquids.

The water-insoluble, water-swellable absorbing polymers 26 of the present invention are not particularly limited in structure, except that the liquid-absorbing polymer material must exhibit minimal solubilization and substantial swelling upon absorption of several hundred times its weight of water. Such liquid-absorbing polymers include substituted and unsubstituted, synthetic and natural polymers. The water-absorbing resins usually are moderately crosslinked and contain hydrophilic moieties such as carboxyl, sulfonate, sulfate, hydroxy, phosphate and/or amino groups and the like.

The water-absorbing polymers 26 useful in the pouches of the present invention also may include substituted or unsubstituted polymers, copolymers or terpolymers of hydrolysis products of starch-acrylonitrile graft polymers; crosslinked carboxymethylcellulose; crosslinked polyacrylamides; and polymethacrylamides; crosslinked polyacrylates; polyvinyl alcohols; polyacrylonitriles; polyvinylpyrrolidones; sulfonated polystyrene; hydrolyzed polyacrylamides; polyethylene oxide; sulfated hydroxyethyl polyacrylates; sulfated polyvinyl alcohol; sulfated hydroxypropyl methacrylate; polymers derived from methacryloxy ethyl phosphate; polymerized alpha, beta-unsaturated carboxylic acids and their salts; polymerized aromatic or heterocyclic sulfonic acids, such as 2-vinyl-3-bromobenzenesulfonic acid and 2-sulfo-5-allylfurane; polymerized aliphatic sulfonic acids, such as ethylene sulfonic acid; alginates; carrageenen; proteins, such as gelatin, and casein; crosslinked gum arabic; agar; gum ghatti; crosslinked polysaccharide; acrylic or methacrylic acid graft copolymers; starch-acrylic acid graft copolymers; hydrolyzed starch-methyl methacrylate graft copolymers; crosslinked polysaccharide-acrylamide copolymer hydrolyzates, such as crosslinked products of hydrolyzed starch-ethyl acrylate graft copolymer, hydrolyzed starch-methyl methacrylate graft copolymer, hydrolyzed starch-acrylonitrile graft copolymer and hydrolyzed starch-acrylamide graft copolymer; crosslinked alkyl acrylate or methacrylate-vinyl acetate copolymer hydrolyzates, such as crosslinked products of hydrolyzed ethyl methacrylate-vinyl acetate copolymer and hydrolyzed methyl acrylate-vinyl acetate copolymer; crosslinked starch-acrylonitrile-acrylamide-2-methylpropanesulfonic acid graft copolymner hydrolyzates; crosslinked starch-acrylonitrile-vinyl-sulfonic acid graft copolymer hydrolyzates; polyfumaride; polyvinylbenzyltrimethylammonium chloride; crosslinked polymers of alpha, beta-unsaturated acids containing a plurality of carboxyl groups, such as fumaric acid and itaconic acid; and the like. Suitable polymers are also disclosed in this assignee's U.S. Patent Nos. 4,618,631; 4,654,393; 4,677,174; and 4,708,067, which patents are hereby incorporated by reference.

The liquid-absorbing polymers useful in the present invention additionally include nonhydrophilic monomers, such as styrene, copolymerized with the hydrophilic monomers, as long as the water-absorption characteristics and swellability of the resulting resin is sufficiently large to allow the resin to function as a fluid loss control agent. The polymers useful in the present invention can be used alone or in admixture, and can be surface treated by various agents, such as polyquaternary amines, to improve the water-absorption, water-retention, organic liquid-absorbing qualities, and water-swellability properties of the polymer. In addition, absorbent fillers such as cellulose pulp, talc, diatamaceous earth, unsaturated comonomers, vermiculite, and partially saponified polyacrylate polymers may be used in amounts of about .1 to about 50 percent by weight of the absorbent material 26.

To achieve the full advantage of the present invention, crosslinked homopolymers of acrylic acid or methacrylic acid, or their salts; crosslinked homopolymers of acrylic acid or methacrylic acid or their salts, synthesized in the presence of a styrene-maleic anhydride resin; or crosslinked polymers derived from acrylic acid or methacrylic acid copolymerized with styrene, wherein the polymers can be synthesized from a monomer solution either including a styrene-maleic anhydride resin or lacking a styrene-maleic anhydride resin, can be admixed with a water-swellable bentonite to provide an efficient drilling fluid loss control agent.

To further improve the fluid loss capabilities of the bentonite and water-absorbing polymer mixture, the water-absorbing polymer is surface treated with a polyquaternary amine before mixing the water-absorbing polymer with the bentonite. The improved water-absorption and water-retention characteristics realized by copolymerizing acrylic acid with styrene is fully disclosed in U.S. Patent No. 4,677,174 and hereby incorporated by reference. Similarly, the improved water-absorption and water-retention characteristics achieved by polymerizing acrylic acid or methacrylic acid, and optionally, styrene in the pre sence of a styrene-maleic anhydride resin is disclosed in U.S. Patent Application Serial No. 067,233 filed June 25, 1987, which application is incorporated herein by reference. The benefits achieved by surface treating the polymers with a polyquaternary amine are disclosed in U.S. Patent No. 4,755,562, also hereby incorporated by reference.

The thin, flexible, fluid-permeable, non-woven/woven water-permeable fabric or polymeric sheets 28 are chosen from a variety of different materials. One such material is sold by the T.H. Dexter Company of Windsor Locks, Connecticut and is characterized as a two faced, light-weight, permeable, heat-sealing tissue comprising a blend of thermoplastic fibers, cellulose pulp and other cellulose fibers.

Typical properties of a grade 2588 material sold by this company include a weight of 24.6 grams per square meter and an air-permeability of 460 liters per minute, per hundred square centimeters, at 12.7 milimeters of water vapor pressure. Such material has excellent wet strength and dry strength. When the tensile strengths are measured in grams per 25 millimeters, fabric sheets 28 having a dry strength ranging between 5300 to 1990 grams per 25 millimeters and a wet strength of 525 grams per 25 millimeters have been utilized effectively. When wetted with aqueous solutions, the grade 258 woven/non-woven fabric sheets 28 have a pH of 5.2, and the sheet material has been tested in accordance with, and meets the specifications of, the United States Food and Drug Administration, Extraction Tests for Food Packaging Materals in Contact with Aqueous Foodstuffs as set forth in 21 C.F.R. 176.170.

In general, the sheets 28 of fluid-permeable fabric or polymer are thin, light and flexible, and for heat seal applications, contain a percentage of thermoplastic fibers. When the thermoplastic fibres are subjected to heat and pressure between pinch rolls, the fibers on opposite folded over portions of the sheet 28 are bonded along the three (3) heat seal lines 30 which border peripheral edges of the pouch 20.

In accordance with an embodiment of the present invention, the fabric sheets 28 also contain other fibers which are fluid or liquid absorptive, such as cellulose fibers, and/or fibrous forms of the super-liquid-absorbing-polymers previously described herein. By providing the sheets 28 with non fluid-absorbing heat sealable thermoplastic fibers, having excellent wet strength characteristics, along with a mixture of about 5-50% by weight liquid-absorbing-fibers, these fabric sheets are capable of directly absorbing liquids from the exterior of the pouch 20 and also passing most of the liquid inwardly through the permeations to be picked up and contained by the fluid-swellable, super-liquid-absorbing polymer material 26 of the inner layer.

As illustrated in FIGS. 3 and 4, when a liquid 24 is absorbed by contact with the pouch 22, the liquid flows through the permeations in the outer fabric sheet 28 and is absorbed both by the liquid absorbant fibrous material in the outer fabric sheet material and by the super-liquid-absorbing polymer material 26 between the outside layers which swells greatly in size as illustrated in the drawing. Because the super-liquid-absorbing polymer 26 is capable of absorbing substantially all of the liquid brought in through the permeations of the outer fabric sheet material 28 of the pouch 20 and because the outer fabric sheet material 28 contains thermoplastic fibers, the outer surface of the pouch 20 in contact with a human or animal or other surface, does not tend to wet the contacting surface or result in a cold clammy feeling against the human skin. This characteristic makes the pouches 20 especially suitable for use in sanitary napkins, bandages, incontinent garments, diapers and the like. Dependent on the amount and type of liquid that is absorbed, the pouches may grow in volumetric size up to 60 to 800, typically 50 to 100 times, and when aqueous solutions or water is absorbed, the ratio of dry volume to wet volume may commonly reach six to eight hundred. Liquid is absorbed not only in the inner layer of super-liquid-absorbing-polymer 26, but is also absorbed and contained in the hydrophyllic fibrous material that is optionally included on the outer fabric sheet 28 forming the outside surface of the pouch 20. This fabric sheet acts as a check valve in permitting passage of fluid to be absorbed inwardly into the interior of the pouch 20 but generally preventing the liquids absorbed from flowing back outwardly and contacting the adjacent surfaces in the immediate environment of the pouch.

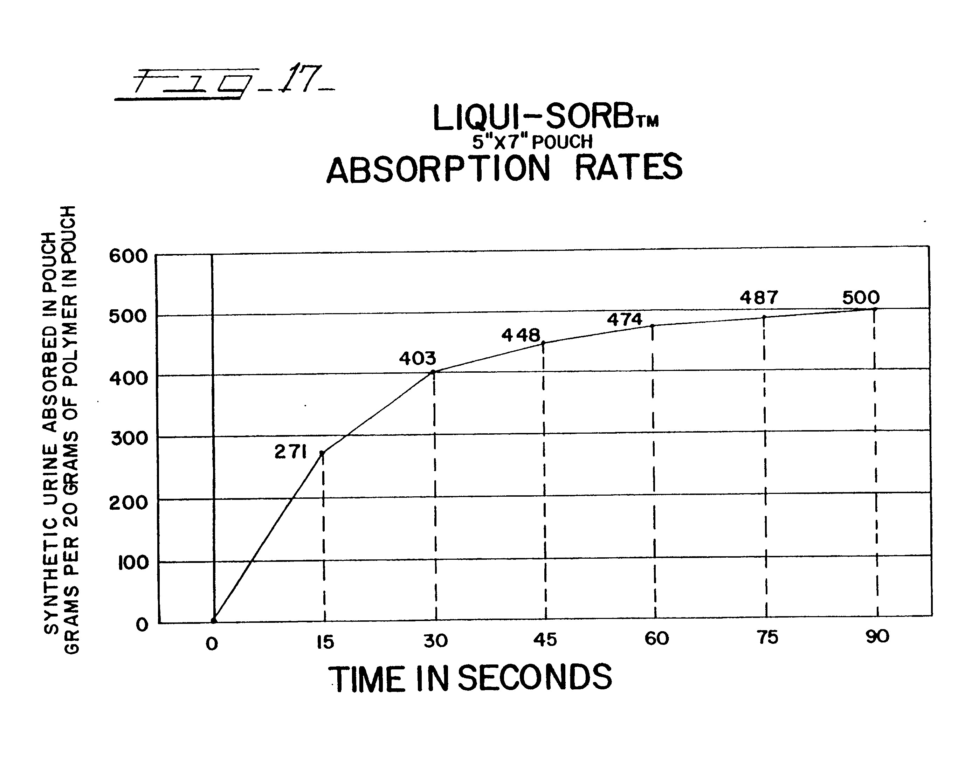

Referring now more particularly to FIGS. 5-8, therein is illustrated a pouch 20A in accordance with the features of the present invention which differs from the pouch 20 as previously described in that a pair of rectangular sheets 28 are provided rather than a single sheet folded in half along the fold line 29. Accordingly, in the pouch 20A, a linear heat seal 30 is provided along all four (4) sides of the sheets 28.

Referring now more particularly to FIGS. 9 through 13, therein is illustrated another embodiment of a pouch constructed in accordance with the features of the present invention and referred to generally by the reference numeral 120 in FIG. 11. The pouch 120 includes a pair of opposite, outside layers of fluid-permeable, thin, flexible, woven/non-woven, resilient, fabric or polymeric sheets 128 which preferably are cut to a rectangular shape, as illustrated. During manufacture of the pouch 120, the lower sheet 128 is positioned directly below a dispensing nozzle or sifter (not shown) which discharges a thin layer of super-liquid-absorbing-polymer material 126 in granulated, particulate or powdered form to a desired, substantially uniform thickness level, e.g., 1 to 20 mils, on the lower fabric sheet. Thereafter an upper fabric sheet 128 is placed above the layer of super-liquid-absorbing polymer 126 and the upper and lower fabric sheets 128 are then compressed toward one another under suitable pressure to form a unitary, thin, multi-layered, sandwich 133 as shown in a cross-sectional view in FIG. 10. The same type of sandwich sheet material 133 may also be used in place of the outer fabric polymeric sheets 128 or the sheets 28 of the previously described embodiment. Similarly, super-liquid-absorbing-polymer material 126 may be chosen from the same group of polymer materials 26 as previously set forth herein.

It has been found that by tightly pressing the permeable, fabric or polymeric sheets 128 against opposite sides of a layer of super-liquid-absorbing polymer material 126, that an integral sandwich sheet 133 (FIG. 6) is formed which is flexible, yet liquid permeable so that liquid from the exterior may readily penetrate and be absorbed, both by any liquid absorbing fibers that may be present in the outer fabric sheets 128 and by the granular, particulate or powdered super-liquid-absorbing polymer material 126 sandwiched therebetween, as in the pouchs 20 and 20A of the previous embodiments.

The pouch 120 of FIG. 11 is formed by folding over a rectangular shaped sheet of sandwich material 133 along a bisecting fold line 129 to form a rectangular or square shape, as illustrated. Once the fold along one edge 129 is accomplished, three lines of heat sealing 130 are provided along the remaining three peripheral edges of the rectangular shaped pouch 120. The resultant construction provides an open air space 131 between the folded over sandwich layers 133 as illustrated in FIGS. 12 and 13. This open space 131 accommodates an even greater amount of swelling action while eliminating the need for a fourth line of heat sealing 130 along the fold line 129.

In accordance with the present invention, a sandwich sheet 133 constructed in accordance with the embodiment as shown in FIG. 10 is effectively utilized in the packaging and retail marketing of foodstuffs including meats such as beef, pork, poultry and fish by placing the sheet 133 beneath the foodstuff in an ordinary window package having a covering window of transparent material. In many instances, such foodstuffs tend to secrete or release unsightly liquids such as blood and oil or grease many hours or even days after the initial packaging of the product and these liquids often render the package unappetizing to a potential buyer. By the placement of a sandwich sheet 133 containing super-liquid-absorbing-polymer material beneath the foodstuff in a package, the liquids secreted from the foodstuff are rapidly absorbed within the super polymer liquid absorbing material 126 and discoloration is eliminated or greatly minimized.

Referring now to FIGS. 14, 15 and 16, therein is illustrated yet another important embodiment of a pouch in accordance with the present invention referred to generally by the reference numeral 220. The pouch 220 includes an inner layer of super-liquid-absorbingpolymer 226 sandwiched between a pair of flexible, permeable woven/non-woven fabric sheets 228 of the type having high wet strength and resiliency as described in connection with the fabric sheets 28 and 128 hereinbefore described.

The fabric sheets 228, however, do not contain a substantial percentage of heat sealable, thermoplastic fibers but instead are made from fibrous materials having only a fluid-absorbency characteristic, as previously described in connection with the sheets 28 and 128. The outer flexible sheets 228 of liquid-absorbing material are pressed against the deposited layer of granular or particulate super-liquid-polymer 226 to provide an integral sandwich sheet 233 which is then cut into rectangular shape or other suitable shapes in a size to be inserted within a pocket or envelope, of slightly larger size envelope, formed with two outer sheets of fluid-permeable, thin, flexible woven/non-woven fabric or polymeric sheet material 228A.

These outer sheets may contain heat-sealable thermoplastic fibers or may be formed entirely of thermosetting or liquid-absorbtive type fibers. If thermoplastic fibers are not present in the sheets 228A, the sheets are joined together along peripheral edges by means of compatible adhesive layers 230A (FIGS. 15 and 16). The adhesive comprises a liquid inosluble resin such as a polyester, epoxy or acrylic adhesive resin. After the rectangular sandwich insert 233 is inserted into the pocket between the outer flexible fabric sheets 228A, as illustrated in FIG. 14 by the arrow "A", the upper (fourth) edge of the flexible fabrics are joined either by heat sealing together or by an adhesive layer or strip.

The novel pouch 220 provides a low cost and highly efficient, liquid absorbing pouch having a substantially uniform thickness of super-liquid-absorbing polymer material 226 over the entire area within the pocket between the outer sheets 228A, regardless of whether the pouch is disposed in a horizontal or vertical position. Because the inner layer of super-liquid-polymer 226 is initially pressed in place be tween the two holding layers 228 and thereby formed into an integral sandwich sheet 233, the super-liquid-absorbing-polymer 226 cannot drop to the lower end of the pouch 220 when the pouch is disposed vertically. Moreover, after insertion of the sandwich sheet 233 between the outer sheets of flexible fabric 228A, friction helps to retain the sheets in a flat condition. Before insertion of the inner sandwich layer 233, the outer sheets 228A are secured together around three peripheral edges, either by adhesive material 230A or by lines of heat seals 230 when the outer fabric sheets 228A contain heat-sealable thermoplastic fibers. After insertion of the inner sandwich layer 233, the fourth edge of the pouch 230 is sealed by a line of adhesive of heat sealing.

Although the present invention has been described in terms of several preferred embodiments, it is intended to include those equivalent structures, some of which may be apparent upon reading this description, and others that may be obvious after study and review.

## Claims

1. A pouch (20,20A,120,220) for absorbing liquids characterised by comprising:
an outside sheet (28,128,228) of fluid-permeable, flexible, material,
an intermediate layer of liquid-swellable, liquid-absorbing polymer material (26,126,226) deposited in a thin layer between folded over portions of said outside fabric sheet for absorbing fluid passing inwardly through the outside sheet material, and
means for securing said folded over portions of said outside sheet together around peripheral edges thereof for containing said liquid-swellable, liquid-absorbing polymer (26,126,226).

2. A pouch as claimed in claim 1, characterised in that the outside sheet (28,128,228) includes fibres of resilient thermoplastic, essentially non-absorbing material and fluid-absorbing fibrous material.

3. A pouch as claimed in claim 1 or 2, characterised in that said intermediate layer comprises a discrete, unitary fluid absorbing element having a thin layer of said liquid-absorbing polymer (226) pressed between a pair of liquid-absorbing fibre-containing thin sheets (228) of fluid-pervious fabric secured thereto forming a discrete sandwich-like fluid-absorbing element that is disposed between said folded over portions of said outside sheet (228A) to cover substantially all of the area thereof contained within said peripheral edges.

4. A pouch as claimed in any one of the preceding claims, characterised in that said securing means comprises a heat seal (30,130,230) between said folded over portions of said outside sheet formed adjacent said peripheral edges thereof.

5. A pouch as claimed in any one of the preceding claims, characterised in that said securing means comprises a strip of adhesive material applied between said folded over portions of said outside sheet along said peripheral edges thereof.

6. A pouch as claimed in claim 5, characterised in that said adhesive material is not water-soluble.

7. A pouch as claimed in any one of the preceding claims, characterised in that said intermediate layer comprises said liquid-absorbing polymer in powder form intermixed with fibrous cellulose particulate material.

8. A pouch as claimed in any one of the preceding claims, characterised in that said liquid-absorbing polymer comprises a water-absorbent, cross-linked polyacrylate; a cross-linked neutralized polyacrylic acid resin surface treated with a polyquaternary amine; or a high molecular weight, acrylic polymer containing hydrophilic carboxylate groups which form a gel or fluff on contact with said body fluid.

9. A pouch as claimed in any one of the preceding claims, characterised in that said outside sheet includes fibrous wicking material; and said liquid-absorbing polymer contains a material selected from the group consisting of cellulose, talc, diatamaceous earth, partially saponified polyacrylate, ethylenically unsaturated comonomers, and vermiculite.

10. A pouch for absorbing liquids, characterised by comprising:
an inner layer of liquid swellable, liquid-absorbing polymer material (26,126,226) sandwiched between a pair of thin, flexible, water-permeable sheets (28,128,228) for the passage of liquid from the exterior of said pouch inwardly to contact and be absorbed by said liquid-swellable, liquid-absorbing polymer in said inner layer;
said liquid-swellable material comprising a layer of particulate polymer having voids between polymer particles for the passage of liquid to be absorbed; and
said water-permeable outer sheets comprising resilient fabric or polymeric sheet material having both non-liquid absorbing fibres and liquid-absorbing fibres for drawing liquid into said inner layer from outside said pouch in contact therewith.

11. A pouch as claimed in claim 10, characterised in that
said sandwiched together outer sheets and said inner layer are folded over along a mid portion thereof leaving peripheral edges extending between opposite ends of a fold line; and
means for securing together said outer sheets along said peripheral edges to contain said inner layer between end portions on opposite sides of said fold line of a single, outwardly exposed sheet forming an outer enclosure for said pouch.

12. A pouch as claimed in claim 11, characterised in that
said outer sheets and said inner layer are substantially rectangular in shape and said fold line bisects said rectangular shape.

13. A pouch as claimed in claim 12, characterised in that:
said securing means comprises linear heat seals along said peripheral edges established between said non-liquid absorbing fibres of said outer sheets.

14. A pouch as claimed in any of claims 11 to 13, characterised in that
said securing means comprises lines of non-liquid absorbing adhesive material along said peripheral edges between said outer sheets.

15. A pouch as claimed in any one of claims 10 to 14, characaterised in that
said inner layer includes a thin layer of water-absorbing polymer material in powder form pressed between said outer sheets.

16. A pouch as claimed in claim 15, characterised in that the polymer material of said inner layer is sized to pass through a 20 mesh screen and be retained on a 100 mesh screen.

17. A pouch as claimed in any one of claims 10 to 16, characterised in that said inner layer of material includes about 1% to about 50% by weight of the inner layer of a·fibrous, cellulosic material.

18. A pouch as claimed in any one of claims 10 to 17, characterised in that said water-absorbing polymer includes a high molecular weight, acrylic polymer containing hydrophilic carboxylate groups which form a gel or fluff upon contact with said liquid.

19. A pouch as claimed in any one of claims 10 to 18, characterised in that at least one of said fabric sheets comprise a two-phase, lightweight, air-permeable, heat seal tissue of blended thermo-plastic fibres, abaca pulp and cellulose fibres.

20. A pouch as claimed in any one of claims 10 to 19, characterised in that at least one of said fabric sheets has a weight of approximately 20-30 grams per square metre.

21. A pouch as claimed in any one of claims 10 to 20, characterised in that at least one of said outer sheets has a tensile strength when completely wetted with water in a range of 499-600 grams per 25 millimetres.

22. A pouch as claimed in any one of claims 10 to 21, characterised in that said liquid-absorbing polymer material has a capacity for absorbing about 50 to about 600 grams of saline solution, blood, urine and/or other body fluids per gram of polymer material.

23. A pouch as claimed in any one of claims 10 to 21, characterised in that said liquid-absorbing polymer material has a capacity for absorbing approximately 600-800 grams of aqueous liquid per gram of polymer material.

24. A method of packaging foodstuffs and the like having liquids released therefrom, characterised by comprising:

forming a layer of liquid swellable, super-liquid-absorbing polymer (26,126,226) sandwiched between a pair of thin, flexible, water-permeable, outer sheets (28,128,228) for the passage of liquid from outer sheets into contact with said polymer, and

placing a surface of said foodstuff in contact with one of said outer sheets for absorption of liquid released from said foodstuff into said polymer.

25. A method of packing foodstuffs and the like, having liquid released therefrom, characterised by comprising placing a surface of said foodstuff into contact with an outside sheet of a pouch as claimed in any one of claims 1 to 23.

26. A method as claimed in claim 24 or 25, characterised in that one of said outer sheets has a wet strength of at least about 200 grams per 25 millimetres.

27. A method as claimed in any one of claims 24 to 26, characterised in that said liquid-swellable, liquid-absorbing polymer has a capacity for absorbing at least about 50 grams of said liquid per gram of polymer.

28. A method as claimed in any one of claims 24 to 27, characterised in that said polymer material is in powder form deposited in a thin, uniformly dispersed layer pressed between said outer sheets.

29. A method as claimed in any one of claims 24 to 28, characterised in that said outer sheets comprise a two-phase, lightweight, air-permeable, tissue of blended thermo-plastic fibres, abaca pulp and cellulose fibres capable of passing a USFDA extraction test mandated in 21 C.F.R. §176.170.

30. A method as claimed in any one of claims 24 to 29, characterised in that said liquid-swellable, liquid-absorbing polymer material has a capacity to absorb at least about 50 times its own weight in liquids released from said foodstuffs.

FIG-1-

FIG-2-

FIG-2A-

FIG-3-

FIG-4-

FIG-5-

FIG-6-

FIG-7-

FIG-8-

*FIG_9_*

126

128

128

10

10

*FIG_10_*

128

126

133

128

*FIG_11_*

130

130

128

129

13

13

12

12

130

120

*FIG_12_*

126

128

128

131

129

*FIG_13_*

128

126

130

128

131

_FIG_14_

_FIG_15_

_FIG_16_

FIG. 17.

LIQUI-SORB™
5"x7" POUCH
ABSORPTION RATES

TIME IN SECONDS

SYNTHETIC URINE ABSORBED IN POUCH
GRAMS PER 20 GRAMS OF POLYMER IN POUCH